# EUROPEAN PATENT APPLICATION

(11) **EP 2 604 109 A1**
(43) Date of publication of application: **19.06.2013**
(21) Application number: 11816513.3
(22) Date of filing: 21.01.2011
(51) Int. Cl.: A01K 67/027, G01N 33/15, A61B 5/0476, A61P 25/00

(54) **MODEL MOUSE OF ATTENTION DEFICIT HYPERACTIVITY DISORDER, METHOD FOR INVESTIGATING EFFECTS FOR PREVENTING AND ALLEVIATING ATTENTION DISORDER USING THE MODEL MOUSE, AND METHOD FOR TREATING ATTENTION DISORDER BY SUPPRESSING T-TYPE CALCIUM CHANNEL**

(30) Priority: 13.08.2010 KR 20100078159
(71) Applicant: Korea Advanced Institute Of Science And Technology, Daejeon 305-701 (KR)
(72) Inventor: KIM, Daesoo, Daejeon 305-741 (KR); KIM, Jeongjin, Daejeon 305-770 (KR)
(74) Representative: Grünecker, Kinkeldey, Stockmair & Schwanhäusser
(86) International application number: PCT/KR2011/000433
(87) International publication number: WO 2012/020897

(57) **Abstract**

The present invention relates to an Attention deficit and hyperactivity disorder(ADHD) mouse model and a screening method for a composition for the prevention and treatment of attention deficit disease. The mouse model constructed by inserting cobalt wire in the right side of the frontal lobe is very useful as the ADHD mouse model, and the said ADHD mouse model can be effectively used for the screening of an agent for the treatment of attention deficit disease since the mouse demonstrated brain wave with low frequency, hyperactivity, and cognitive impairment, which are characteristic symptoms of attention deficit disease, when ethosuximide, one of T-type calcium channel blockers, was administered. Therefore, the composition comprising a T-type calcium channel blocker was also confirmed to be effectively used for the prevention and treatment of attention deficit disease.

<110>    Korea Advanced Institute of Science and Technology

<120>    Attention deficit hyperactivity disorder model animal method for
         evaluating prevention and alleviation of attention deficit
         disease and method for prevention and treatment of attention
         deficit disease by inhibiting T type calcium channel

<130>    10fpo-11-07

<150>    KR 10-2010-0078159
<151>    2010-08-13

<160>    2

<170>    KopatentIn 1.71

<210>    1
<211>    75
<212>    DNA
<213>    Artificial Sequence

<220>
<223>    Cav3.1 shRNA


<400>    1
cggaattccg ggaagatcgt agatagcaaa ttcaagagat ttgctatcta cgatcttctt        60

tttgatatct agaca                                                         75


<210>    2
<211>    74
<212>    DNA
<213>    Artificial Sequence

<220>
<223>    Cav3.1 control shRNA


<400>    2
ggaattccgg gtaagtgaac tgacaagaat tcaagagatt cttgtcagtt cacttacttt        60

ttgatatcta gaca                                                          74

## Description

### BACKGROUND OF THE INVENTION

### 1. Field of the Invention

The present invention relates to an attention deficit hyperactivity mouse model constructed by implanting cobalt wire, a method for screening a composition for the prevention and treatment of attention deficit disease, and a method for the treatment of attention deficit disease using the same.

### 2. Description of the Related Art

Attention deficit and hyperactivity disorder (referred as ADHD hereinafter) is a kind of mental disorder frequently observed in the children before and after school-age, and major symptoms of the disease are inattention, hyperactivity, and impulsivity. ADHD accompanies many psychosocial disorders including learning disorder, and can be a reason of delinquency or deviant behavior in adolescence and drug addiction and crime in adulthood (Y. B. Lee, J. S. Shin, Korean journal of family social work, 2000; G. D. Kewley, British Medical Journal, 1998; L. B. Silver, Attention-deficit hyperactivity disorder, Washington, DC.: American Psychiatry Press, Inc. 1992).

Attention deficit and hyperactivity disorder is attributed to many different reasons such as genetic factors (R. A. Barkley. Attention deficit hyperactivity disorder: A handbook of diagnosis and treatment. New York: Guilford Press. 1990), biochemical factors counter-acting resulted from taking lead and instant food additives (G. David, J. Neal, Abnormal Psychology. John Wilry & Sons. 1976), selection problem of transmitting stimuli to the brain (H. S. Lee, Journal of Emotional & Behavioral Disorders, 16), and environmental factors exemplified by relationship between parents and children and social position of parents, smoking and alcohol drinking during pregnancy, etc. However, neurobiological factors seem to be more important factors than psychosocial factors. Thus, studies are actively going on to identify biological reasons for ADHD and to develop an agent to treat the disorder.

In the studies of ADHD, neuroanatomical abnormality was found in the frontal lobe, and abnormality in neurotransmitter was also found in the central nervous system in the aspect of neurobiochemistry, and also neurobiological abnormalities were detected in the prefrontal cortex, in the thalamus, and in the dopaminergic mesocortical projection as well. ADHD is highly suspected as a kind of nonuniform disorders that is caused rather by abnormalities in interrelation among many brain regions taking responsibility in high cognitive function than by a single disorder in neuron development (Y. Frank, S. G. Pavlakis, Pediatric Neurology., 2001; B. N. Kim et al., The Korean Journal of Neurokiatric Association, 2000). According to the recent structural and functional brain image studies with young ADHD patients, pathophysiology of ADHD seems to be related to dysfunction of the fronto-striatal tract and MPH (Methylphenidate) might cause functional changes of dopamine system in that area (M. H. Teicher, C. M. Anderson et al., Nat. Med., 2000; J. B. Schweitzer, D. O. Lee et al., Neuropsychopharmacology, 2003; E. Y. Oh, I. Hwang et al., J. Korean Soc. Biol. Ther. Psychiatri., 2003). However, the exact reason causing ADHD has not been identified and the above-mentioned reasons are presumed to be co-working to develop ADHD (H. Mang, H. Chung, The Journal of Elementary Education., 2005).

Symptoms of ADHD are related to noradrenergic neuroregulation that controls neural circuit in the frontal lobe, in addition to dopaminergic neuroregulation. That is, behavioral symptoms of ADHD are resulted from the unbalance of noradrenergic neuroregulation and dopaminergic neuroregulation, according to the previous reports. In general, it is presumed that the decrease of influence of catecholamines regulating glutamatergic neurons and GABA(g-aminobutyric acid)ergic neurons is the reason (T. Sagvolden, T. Xu. Behav. Brain Funct. 4, 2008; E. B. Johansen, P. R. Killeen et al., Behav. Brain Funct. 3, 2007).

The most common agent to treat ADHD is a CNS stimulant, which is exemplified domestically by Ritalin and Methylphen comprising Methylphenidate as an active ingredient. These drugs are known to have short term effect on relieving such symptoms as inattention, hyperactivity, and impulsivity, but long term effect has not been confirmed (S. Kim, D. Ahn, Y. Lee, J. Korean Neuropsychiatr. Assoc. 4, pp683-699, 1998).

Attention deficit disease like ADHD is exemplified by schizophrenia, frontal lobe epilepsy, and autism, etc. Common symptoms of these diseases are hyperactivity and oscillation with low frequency in the frontal lobe (Callicott et al., 2000; Manoach et al., 2000, Barry et al., 2003; Wienbruch et al., 2003; Verkhliutov et al., 2006; Yoon et al., 2008; Kumari et al., 2009).

Ethosuximide is a T-type calcium channel blocker, which has been used as an agent to treat epilepsy. In addition, according to the "METHOD FOR TREATMENT OF ANXIETY DISORDER BY REGULATING T-TYPE CALCIUM CHANNEL (Korean Patent No. 10-09582910000), the use of a composition comprising ethosuximide for the treatment of anxiety disorder has been reported. However, the use of ethosuximide as an agent to treat attention deficit has not been disclosed yet. The said anxiety disorder is the disease having problems of memory formation of fear in the frontal lobe and amygdale. Such functional abnormality is frequently found in phobic disorder, generalized anxiety disorder, obsessive compulsive disorder, post-traumatic stress disorder, somatoform disorder, dissociative disorder, and factitious disorder, etc. Anxiety disorder is developed by a structural problem in the brain particularly in the area that is responsible for emotion and memory, which is distinguished from attention deficit and hyperactivity disorder. The preset invention is useful for the understanding of mechanism of emotion and memory formation and for the development of a novel drug for the treatment of such disorder.

The present invention is based on the understanding of ADHD. That is, the foundings of the present inventors are based on the understanding not of emotion and memory formation but of generative process of attention. Despite huge efforts made by many researchers to explain generative process of attention, there is not much to show on this. Attention deficit is general symptom found not only in ADHD (attention deficit hyperactivity disorder) but also in schizophrenia, frontal lobe epilepsy, and autism. Attention deficit is a huge barrier for a person to be adapted socially. Hyperactivity and stereotypy observed in ADHD are also shown in schizophrenia. Low frequency brain wave observed in the frontal lobe is also observed in the said two diseases. According to many clinical reports, the aspects of the above two diseases demonstrated a lot like same. Based on the above observations and study results, it can be presumed that ADHD shares the same mechanism of attention deficit with schizophrenia.

The present inventors constructed an animal model by inserting cobalt wire in the brain of the animal and observed electrical excitation with low frequency in the frontal lobe, behavioral disorder, and recognition deficit of the animal model, which are usually observed in attention deficit disease patients. To confirm the specific mechanism of attention deficit, the present inventors constructed T-type calcium channel knockout mouse and animal model wherein T-type calcium channel was removed. As a result, the inventors confirmed that T-type calcium channel is involved in the regulation of hyperactivity and oscillation with low frequency. The present inventors administered Ethosuximide, Zonisamide, and Phyenytoin, the T-type calcium channel blockers, to ADHD mouse model and observed that spike type brain wave was reduced in the frontal lobe. Therefore, the present inventors confirmed that the animal model inserted with cobalt wire could be used effectively as ADHD mouse model and at the same time it could be effectively used for the screening of an agent for the treatment of attention deficit disease. The inventors further confirmed that a composition comprising a T-type calcium channel blocker could be effectively used as a composition to treat attention deficit disease, leading to the completion of this invention.

### SUMMARY OF THE INVENTION

It is an object of the present invention to provide an ADHD mouse model constructed by implanting cobalt wire in the brain of the mouse.

It is another object of the present invention to provide a method for screening of an agent for the treatment of attention deficit disease by using the said ADHD mouse model.

It is further an object of the present invention to provide a composition for the prevention and treatment of attention deficit disease comprising a T-type calcium channel blocker.

To achieve the above objects, the present invention provides an ADHD mouse model constructed by implanting cobalt wire in the brain of the mouse.

The present invention also provides a method for constructing ADHD mouse model comprising the following steps:
1) implanting cobalt wire in the side of the right frontal lobe of mouse brain;
2) suturing the mouse brain after completing the implant of cobalt wire; and
3) selecting those mice showing attention deficit.

The present invention further provides a screening method of an agent for the treatment of attention deficit disease comprising the following steps:
1) administering test compounds or compositions to the ADHD mouse model of the present invention;
2) measuring brain wave of the ADHD mouse model administered with test compounds or compositions of step 1); and
3) selecting the test compound or the composition demonstrating to be effective in reducing abnormal electroencephalogram(EEG)(brain wave) spikes in the ADHD mouse model of step 2) by comparing them with the control group not treated with test compounds or compositions.

The present invention also provides a screening method of an agent for the treatment of attention deficit disease comprising the following steps:
1) administering test compounds or compositions to the ADHD mouse model of the present invention;
2) investigating behavioral disorder or cognitive function of the ADHD mouse model administered with test compounds or compositions of step 1); and
3) selecting the test compound or the composition demonstrating to be effective in recovery of behavioral disorder or cognitive function by comparing the brain wave of the ADHD mouse model of step 2) with that of the control group not treated with test compounds or compositions.

The present invention also provides a composition for the prevention and treatment of attention deficit disease comprising a T-type calcium channel blocker as an active ingredient.

The present invention also provides a health food for the prevention and improvement of attention deficit disease comprising a T-type calcium channel blocker as an active ingredient.

The present invention also provides a feed additive for the prevention and improvement of attention deficit disease comprising a T-type calcium channel blocker as an active ingredient.

The present invention also provides a use of the ADHD mouse model of the present invention for the screening of an agent for the treatment of attention deficit disease.

The present invention also provides a use of a T-type calcium channel blocker for the preparation of an agent for the treatment of attention deficit disease.

The present invention also provides a use of a T-type calcium channel blocker for the preparation of a health food for the improvement of attention deficit disease.

In addition, the present invention provides a use of a T-type calcium channel blocker for the preparation of a feed additive for the improvement of attention deficit disease.

### ADVANTAGEOUS EFFECT

As explained hereinbefore, the mouse model constructed by inserting cobalt wire of the present invention can be effectively used as an ADHD mouse model since this mouse model demonstrates increased electrical excitation with low frequency, hyperactivity, and reduced cognitive function which are characteristic symptoms of attention deficit disease, and can be effectively used for the screening of an agent for the treatment of attention deficit disease showing the said symptoms alike. When ethosuximide, one of T-type calcium channel blockers, was administered to the ADHD mouse model, the number of spikes observed in brain wave was reduced. Therefore, the composition comprising a T-type calcium channel blocker can be effectively used for the treatment of attention deficit disease.

### BRIEF DESCRIPTION OF THE DRAWINGS

The application of the preferred embodiments of the present invention is best understood with reference to the accompanying drawings, wherein:

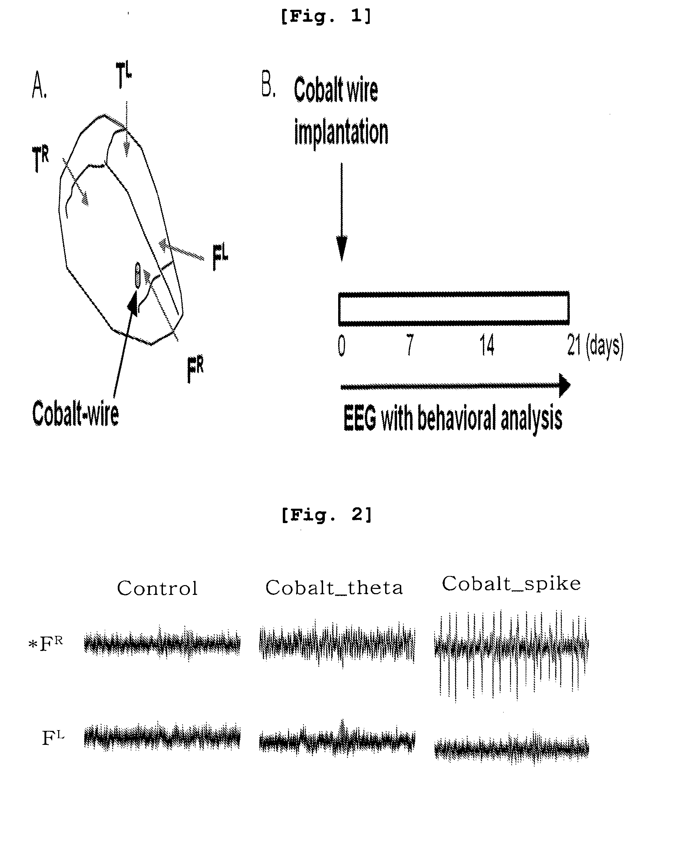

Fig. 1 is a set of diagrams illustrating the construction of an ADHD mouse model and the processes of the experiment:
   Figure 1A is a diagram illustrating the location where cobalt wire and EEG electrode were inserted to construct the ADHD mouse model and for the behavioral analysis;
      Cobalt-wire: Cobalt wire (Alfa Aesar) of 500 *µ*m in diameter was inserted radially (vertical direction to the cortical surface) in the location of anteroposteriorally 2.6 mm, laterally 1.8 mm, and ventrally 1.3 mm in the right dorsolateral frontal lobe;
      F^{R}: right frontal lobe;
      F^{R}: left frontal lobe;
      T^{R}: right temporal lobe; and
      T^{L}: left temporal lobe;
   Figure 1B is a diagram illustrating the investigation period of EEG changes and behavioral analysis after the implant of cobalt wire.
Figure 2 is a set of diagrams illustrating the EEG record of the ADHD mouse model:
   control: the implanted control group;
   cobalt_theta: the brain wave with the frequency of 4 - 7 Hz observed in the mouse implanted with cobalt wire;
   cobalt_spike: the spike type brain wave with the frequency of 1 - 4 Hz observed in the mouse implanted with cobalt wire;
   F^{R}: right frontal lobe; and
   F^{L}: left frontal lobe.

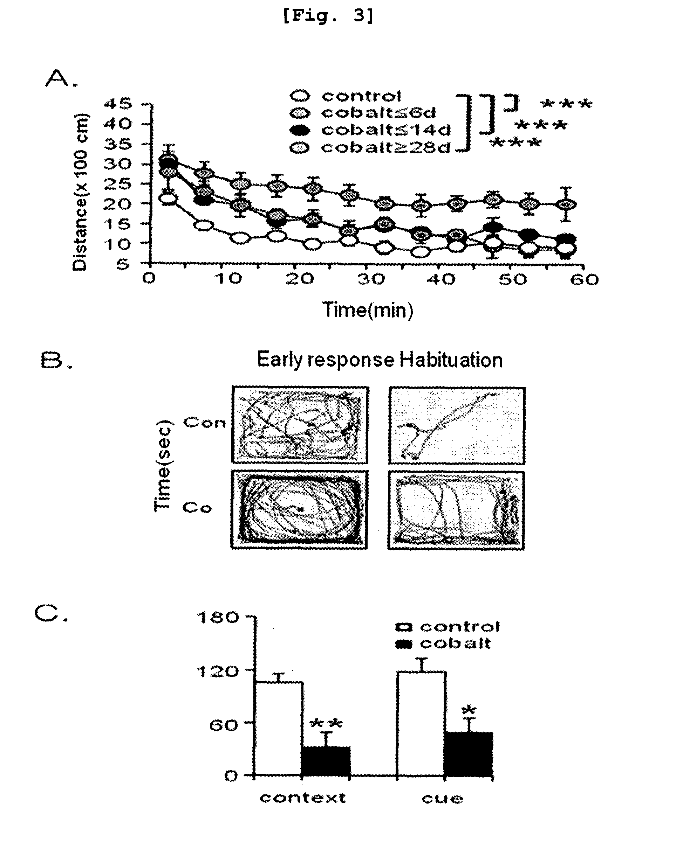

Figure 3 is a set of graphs and photographs illustrating the behavioral changes and cognitive function changes of the ADHD mouse model:
   Figure 3A is a graph illustrating the results of open-field test for the quantification of the behavior of the ADHD mouse model;
      o control: the unimplanted control group;
      ■ cobalt≤6d: the ADHD animal model at 6 days or under the cobalt wire implant;
      ◆ cobalt≤14d: the ADHD animal model at 14 days or under the cobalt wire implant;
      A cobalt≥28d: the ADHD animal model at 28 days or up from the cobalt wire implant;
   Figure 3B is a set of photographs illustrating the results of the video observation of behavior of the ADHD animal model via open-field test;
      Con: the unimplanted control group;
      Co: the ADHD animal model at 28 days or up from the cobalt wire implant;
   Figure 3C is a graph illustrating the results of conditioned fear learning test of the ADHD animal model;
      □ control: the unimplanted control group;
         ■ cobalt: the ADHD animal model at 28 days or up from the cobalt wireimplant;
      context: the group tested if they remembered the place where they learned fear; and
      cue: the group tested if they remembered the sound related to the fear they learned.

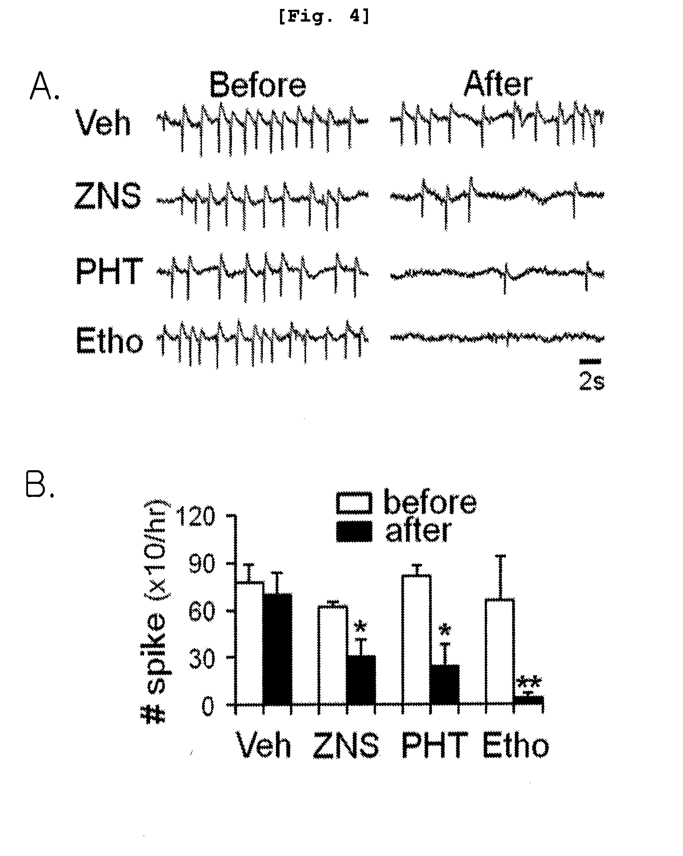

Figure 4 is a set of a graph and a diagram illustrating the observation of the spike type brain waves generated in the ADHD animal model after the administration of ethosuximide, zonisamide, and phyenytoin, the T-type calcium channel blockers:
   Figure 4A is a diagram illustrating the spike type brain waves observed before and after the administration of ethosuximide, zonisamide, and phyenytoin;
      Veh: the control group administered with saline;
      ZNS: the group administered with zonisamide;
      PHT: the group administered with phyenytoin;
      Etho: the group administered with ethosuximide;
      Before: before the administration;
      After: after the administration;
   Figure 4B is a graph illustrating the measurement of the spike type brain waves before and after the administration of ethosuximide, zonisamide, and phyenytoin;
      Veh: the control group administered with saline;
      ZNS: the group administered with zonisamide;
      PHT: the group administered with phyenytoin;
      Etho: the group administered with ethosuximide;
      Before: before the administration; and
      After: after the administration.

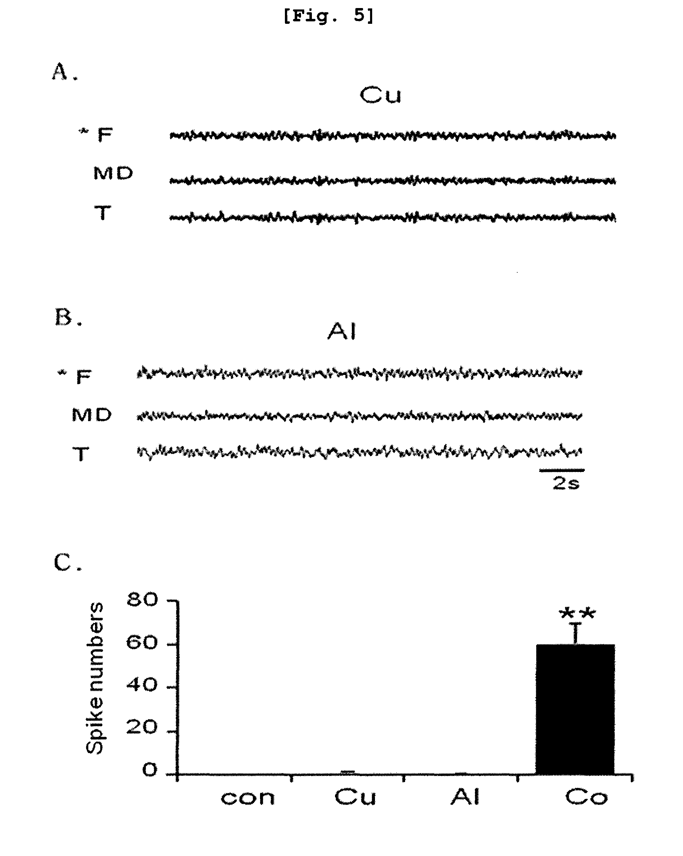

Figure 5 is a set of a graph and diagrams illustrating the observation of spike patterns of the animal models implanted with copper wire, aluminum wire, and cobalt wire:
   Figure 5A is a diagram illustrating the observation of spike pattern of the animal model implanted with copper wire;
      F: right frontal lobe;
      MD: mediodorsal thalamus;
      T: left temporal lobe;
   Figure 5B is a diagram illustrating the observation of spike pattern of the animal model implanted with aluminum wire;
      F: right frontal lobe;
      MD: mediodorsal thalamus;
      T: left temporal lobe;
   Figure 5C is a graph illustrating the spike numbers measured in the animal models implanted with copper wire, aluminum wire, and cobalt wire and in the animal model not implanted with anything;
      Cu: the group implanted with copper wire;
      Al: the group implanted with aluminum wire; and
      Co: the group implanted with cobalt wire.

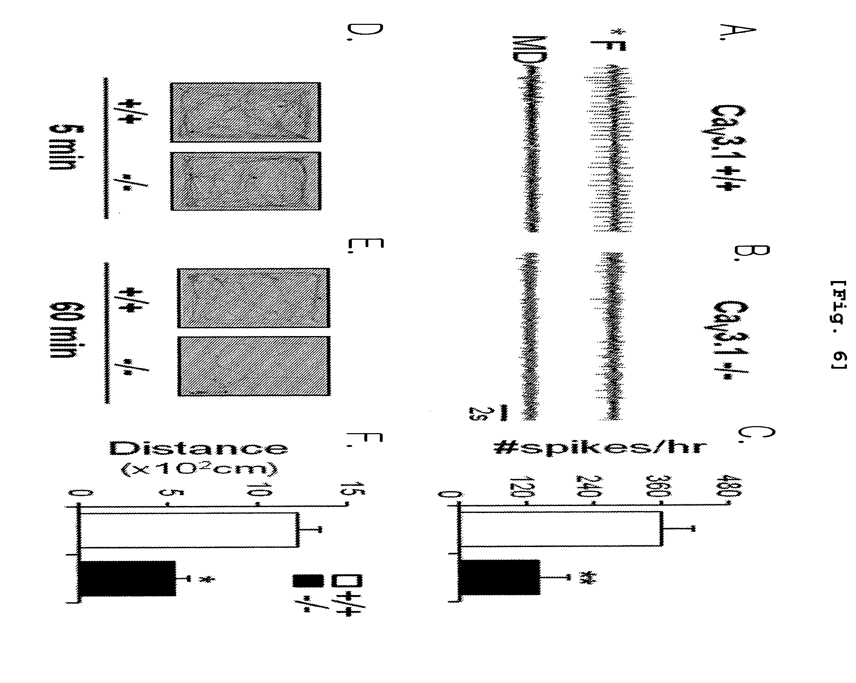

Figure 6 is a set of graphs and diagrams illustrating the changes of behavior and brain waves in the α1G T-type calcium channel knockout animal model:
   Figure 6A is a graph and a diagram illustrating the brain waves observed in the frontal lobe and mediodorsal thalamus of the α1G T-type calcium channel knockout animal model;
      F: right frontal lobe;
      MD: mediodorsal thalamus;
      **: p<0.05;
   Figure 6B is a diagram illustrating the results of the video observation of behavior of the α1G T-type calcium channel knockout animal model via open-field test;
      Caᵥ3.1 +/+: the control group animal model in which α1G T-type calcium channel is not knocked out;
      Caᵥ3.1 -/-: the α1G T-type calcium channel knockout animal model;
      5 min: time from the beginning of open-field test upto 5 minutes; and
      60 min: duration from the time point of 55 minutes from the beginning of open-field test to the time point of 60 minutes from the beginning of open-field test.

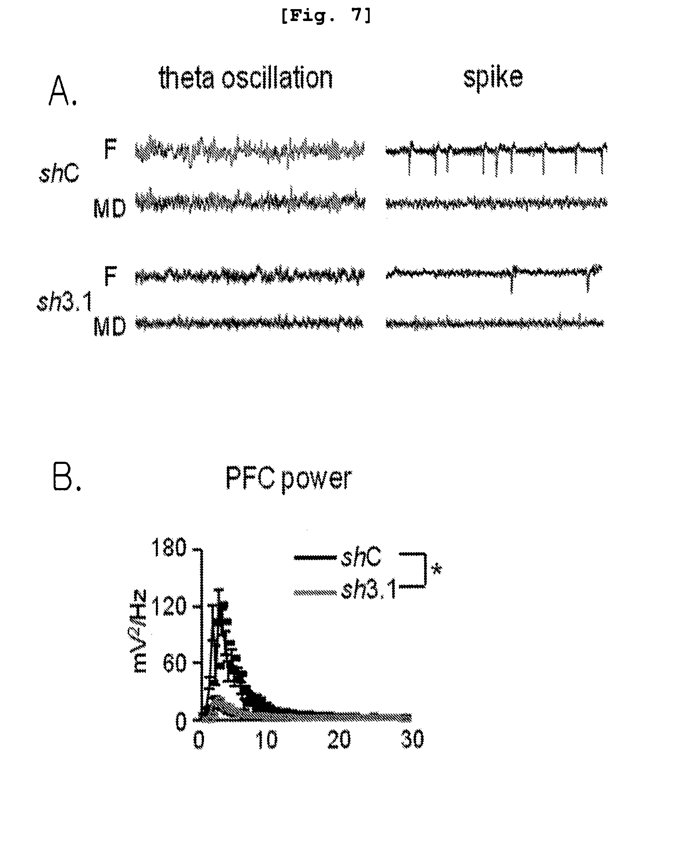

Figure 7 is a set of a graph and a diagram illustrating the brain waves of the animal model in which α1G T-type calcium channel was partially suppressed by inserting lentivirus vector comprising shRNA targeting α1G T-type calcium channel into the mediodorsal thalamus (MD):
   Figure 7A is a diagram illustrating the brain waves observed in the frontal lobe and mediodorsal thalamus of the animal model in which α1G T-type calcium channel was partially suppressed;
      F: right frontal lobe;
      MD: mediodorsal thalamus;
      *shC:* the control group animal model in which α1G T-type calcium channel was not suppressed by inserting lentivirus vector comprising control shRNA not-targeting α1G T-type calcium channel into the mediodorsal thalamus;
      *sh3.1:* the animal model in which α1G T-type calcium channel was partially suppressed by inserting lentivirus vector comprising shRNA targeting α1G T-type calcium channel into the mediodorsal thalamus; and
   Figure 7B is a graph illustrating the huge decrease of brain waves with low frequency in the animal model in which α1G T-type calcium channel was partially suppressed, analyzed by power spectrum analysis.

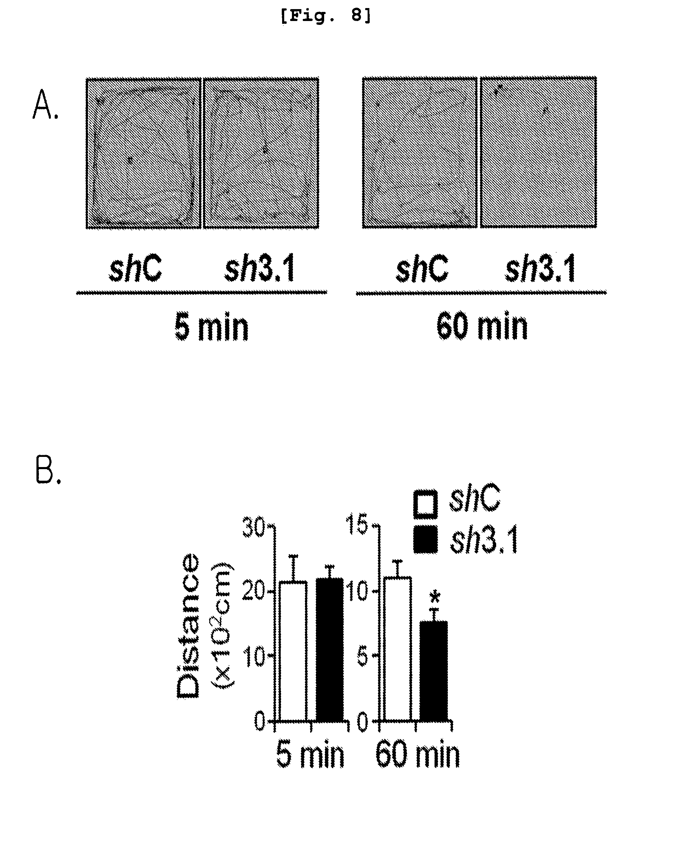

Figure 8 is a set of a graph and a diagram illustrating the behavioral changes in the animal model in which α1G T-type calcium channel was partially suppressed by inserting lentivirus vector comprising shRNA targeting α1G T-type calcium channel into the mediodorsal thalamus (MD):
   Figure 8A is a diagram illustrating the results of video observation of behavior of the animal model in which α1G T-type calcium channel was partially suppressed in the mediodorsal thalamus via open-field test;
      *shC:* the control group animal model in which α1G T-type calcium channel was not suppressed by inserting lentivirus vector comprising control shRNA not-targeting α1G T-type calcium channel into the mediodorsal thalamus;
      *sh3.1:* the animal model in which α1G T-type calcium channel was partially suppressed by inserting lentivirus vector comprising shRNA targeting α1G T-type calcium channel into the mediodorsal thalamus;
      5 min: time from the beginning of open-field test upto 5 minutes; and
      60 min: duration from the time point of 55 minutes from the beginning of open-field test to the time point of 60 minutes from the beginning of open-field test.
   Figure 8B is a graph illustrating the behavior of the animal model in which α1G T-type calcium channel was partially suppressed by inserting lentivirus vector comprising shRNA targeting α1G T-type calcium channel into the mediodorsal thalamus, observed via open-field test;
      *shC:* the control group animal model in which α1G T-type calcium channel was not suppressed by inserting lentivirus vector comprising control shRNA not-targeting α1G T-type calcium channel into the mediodorsal thalamus;
      *sh3.1:* the animal model in which α1G T-type calcium channel was partially suppressed by inserting lentivirus vector comprising shRNA targeting α1G T-type calcium channel into the mediodorsal thalamus;
      5 min: time from the beginning of open-field test upto 5 minutes; and
      60 min: duration from the time point of 55 minutes from the beginning of open-field test to the time point of 60 minutes from the beginning of open-field test.

### DESCRIPTION OF THE PREFERRED EMBODIMENTS

Hereinafter, the present invention is described in detail.

The present invention provides an ADHD mouse model constructed by the following steps:
1) implanting cobalt wire in the side of the right frontal lobe of mouse brain;
2) suturing the mouse brain after completing the implant of cobalt wire; and
3) selecting those mice showing attention deficit.

It is preferred to construct the ADHD mouse model of the present invention according to the steps explained hereinbefore, but not always limited thereto.

To construct the ADHD mouse model, it is preferred to fix the mouse (10 - 20 weeks, C57BL/6J) head on stereotaxic, followed by the insertion of cobalt wire (Alfa Aesar) by using holder, a part of the stereotaxic, but not always limited thereto. In the construction method of the ADHD mouse model, the cobalt wire of step 1) in the diameter of 500 *µ*m is preferably inserted radially (vertical direction to the cortical surface) in the location of anteroposteriorally 2.0 ∼ 3.0 mm, laterally 1.5 - 2.5 mm, and ventrally 1.0 ∼ 2.0 mm in the right dorsolateral frontal lobe, and more preferably inserted in the location of anteroposteriorally 2.0 mm, laterally 1.8 mm, and ventrally 1.3 mm in the right dorsolateral frontal lobe, but not always limited thereto. For the record of EEG, epidural electrode was inserted in the right frontal lobe where the cobalt wire was inserted. Other electrodes were inserted in the left frontal lobe and temporal lobe frontal lobe. Particularly, the location for the insertion in the right frontal lobe was anteroposteriorally 2.8 mm and laterally 0.8 mm and the location in the left frontal lobe was anteroposteriorally 2.8 mm and laterally 0.8 mm. The grounding EEG is preferably inserted in occipital region of the skull, but not always limited thereto.

In a preferred embodiment of the present invention, the present inventors constructed ADHD animal model (see Figure 1). It has been known that epilepsy is induced in the animal model constructed by inserting cobalt wire in the brain (Chang et al., Epilepsy Research, 2004). However, there is no case of using this animal model as ADHD animal model. Therefore, to confirm the possibility of using this mouse model implanted with cobalt wire as ADHD animal model, the present inventors observed brain wave, behavior, and cognitive function, which are characteristically observed in attention deficit disease.

In a preferred embodiment of the present invention, EEG of the mouse model implanted with cobalt wire in the right frontal lobe was analyzed. Brain wave was observed by using EEG electrode inserted in the right and left frontal lobe and temporal lobe of the animal model. As a result, brain wave with low frequency was increased only in the right frontal lobe implanted with cobalt wire. Particularly, theta oscillation of 4 - 7 Hz was first detected and then spikes of 1 ∼ 4 Hz were observed. Such brain wave pattern was not observed in the left frontal lobe not implanted with cobalt wire (see Figure 2).

In a preferred embodiment of the present invention, the changes of behavior and cognitive function of the mouse implanted with cobalt wire were observed. Particularly, cobalt wire was first inserted and open-field test was performed on the 6^{th} day, 14^{th} day, and 28^{th} day from the insertion to monitor behavior. As a result, activity was significantly increased in the mouse implanted with cobalt wire, compared with the control mouse (see Figure 3A). The aspect became more significant as the duration of having cobalt wire inside became longer. More particularly, traces of behavior of the ADHD mouse model were detected to analyze activity. As a result, such stereotypy that the mouse was turning in one direction repeatedly was observed in the mouse implanted with cobalt wire (see Figure 3B). The said stereotypy is most commonly observed in ADHD or schizophrenia (Jones, Comprehensive Psychiatry, 1965; Davids et al., Brain Research Reviews, 2003). To investigate if the mouse implanted with cobalt wire could show memory loss, another common symptom of ADHD, conditioned fear learning test was performed. As a result, memory loss was significant in the ADHD mouse model implanted with cobalt wire, compared with the control group (see Figure 3C). Therefore, since characteristic symptoms of attention deficit disease such as brain wave with low frequency, stereotypy and cognitive impairment were observed in the mouse model implanted with cobalt wire of the present invention, it was confirmed that the mouse model implanted with cobalt wire could be effectively used as an ADHD mouse model.

In a preferred embodiment of the present invention, to alleviate the symptoms of behavioral disorder observed in the ADHD animal, such T-type calcium channel blockers as ethosuximide, zonisamide, and phyenytoin were treated thereto. As a result, the number of spikes observed in the frontal lobe was significantly reduced after the treatment, compared with that of before the treatment (see Figure 4). The said T-type calcium channel blocker is exemplified by ethosuximide, zonisamide, phyenytoin, SUN-N8075, efonidipine, trivalent metal ions selected from the group consisting of Y³⁺, La³⁺, Ce³⁺, Nd³⁺, Gd³⁺, Ho³⁺, Er³⁺, and Yb³⁺, Ni²⁺, U-92032 (7-[[4-[bis(4-fluorophenyl)methyl]-1-piperazinyl]methyl]-2-[(2-hydroxyethyl)amino]4-(1-methylethyl)-2,4,6-cycloheptatrien-1-one), penfluridol, fluspirilene, and valproate, etc. The administration of such T-type calcium channel blocker can alleviate the symptoms of behavioral disorder in the ADHD animal model of the present invention.

In a preferred embodiment of the present invention, to investigate whether or not the mouse not implanted with cobalt wire was useful as the control, the present inventors inserted copper wire or aluminum wire to the right frontal lobe of a mouse instead of cobalt wire. As a result, as shown in Figure 5, spike type brain wave observed in EEG of the mouse implanted with cobalt wire was not observed. This result was consistent with that of the control implanted with nothing. That is, the control group implanted with nothing can be effectively used as the control group to the mouse implanted with cobalt wire and the spike type brain wave observed in the animal model of the present invention has been confirmed to be generated by cobalt wire (see Figure 5).

In a preferred embodiment of the present invention, brain waves and behavioral changes were observed in the α1G T-type calcium channel knockout model (Cav3.1 T-type calcium channel knockout animal model) and the animal model in which α1G T-type calcium channel was partially suppressed by inserting lentivirus vector comprising shRNA targeting α1G T-type calcium channel into the mediodorsal thalamus. As a result, brain wave with low frequency was observed in the frontal lobe of the animal model in which α1G T-type calcium channel was eliminated completely or partially (see Figure 6 - Figure 8). The above results indicate that α1G T-type calcium channel plays an important role in the development of frontal lobe related diseases.

In the mouse implanted with cobalt wire, brain wave with low frequency, behavioral disorder, and cognitive impairment, which are the characteristic symptoms of attention deficit disease, are observed. Therefore, such mouse model can be effectively used as the ADHD mouse model.

The present invention also provides a method for constructing ADHD mouse model comprising the following steps:
1) implanting cobalt wire in the side of the right frontal lobe of mouse brain;
2) suturing the mouse brain after completing the implant of cobalt wire; and
3) selecting those mice showing attention deficit.

To construct the ADHD mouse model, it is preferred to fix the mouse (10 - 20 weeks, C57BL/6J) head on stereotaxic, followed by the insertion of cobalt wire (Alfa Aesar) by using holder, a part of the stereotaxic, but not always limited thereto. In the construction method of the ADHD mouse model, the cobalt wire of step 1) in the diameter of 500 *µ*m is preferably inserted radially (vertical direction to the cortical surface) in the location of anteroposteriorally 2.6 mm, laterally 1.8 mm, and ventrally 1.3 mm in the right dorsolateral frontal lobe, but not always limited thereto. For the record of EEG, epidural electrode was inserted in the right frontal lobe where the cobalt wire was inserted. Other electrodes were inserted in the left frontal lobe and temporal lobe frontal lobe. Particularly, the location for the insertion in the right frontal lobe was anteroposteriorally 2.8 mm and laterally 0.8 mm and the location in the left frontal lobe was anteroposteriorally 2.8 mm and laterally 0.8 mm. The grounding EEG is preferably inserted in occipital region of the skull, but not always limited thereto.

The present invention also provides a screening method of an agent for the treatment of attention deficit disease comprising the following steps:
1) administering test compounds or compositions to the ADHD mouse model of the present invention;
2) measuring brain wave of the ADHD mouse model administered with test compounds or compositions of step 1); and
3) selecting the test compound or the composition demonstrating to be effective in reducing abnormal electroencephalogram(EEG)(brain wave) spikes in the ADHD mouse model of step 2) by comparing them with the control group not treated with test compounds or compositions.

The screening method of an agent for the treatment of attention deficit disease preferably comprises the above steps, but not always limited thereto. In the screening method of an agent for the treatment of attention deficit disease herein, the attention deficit disease is selected from the group consisting of attention deficit hyperactivity disorder, schizophrenia, frontal lobe epilepsy, and autism, but not always limited thereto.

The mouse model implanted with cobalt wire of the present invention demonstrated characteristic symptoms of attention deficit disease such as brain wave with low frequency wave, behavioral disorder, and cognitive impairment. Therefore, this animal model can be effectively used as the ADHD mouse model and for the screening of an agent for the treatment of attention deficit disease as well.

The present invention also provides a screening method of an agent for the treatment of attention deficit disease comprising the following steps:
1) administering test compounds or compositions to the ADHD mouse model of the present invention;
2) investigating behavioral disorder or cognitive function of the ADHD mouse model administered with test compounds or compositions of step 1); and
3) selecting the test compound or the composition demonstrating to be effective in recovery of behavioral disorder or cognitive function by comparing the brain wave of the ADHD mouse model of step 2) with that of the control group not treated with test compounds or compositions.

The screening method of an agent for the treatment of attention deficit disease preferably comprises the above steps, but not always limited thereto. In this screening method of an agent for the treatment of attention deficit disease, the behavioral disorder is preferably investigated by one of the methods selected from the group consisting of open-field test, water maze test, noble object test, and noble recognition test, but not always limited thereto. In this screening method of an agent for the treatment of attention deficit disease, the cognitive function is preferably investigated by one of the methods selected from the group consisting of conditioned fear learning test, pre-pulse inhibition test, and elevated plus maze test, but not always limited thereto. Behavioral disorder and cognitive impairment observed in attention deficit disease indicate any problem in such management functions as planning, impulse control, object management, self-correction of behavior, attention control, and working memory.

The present invention also provides a composition for the prevention and treatment of attention deficit disease comprising a T-type calcium channel blocker as an active ingredient.

In the composition for the prevention and treatment of attention deficit disease, the said attention deficit disease is preferably selected from the group consisting of attention deficit hyperactivity disorder, schizophrenia, frontal lobe epilepsy, and autism, but not always limited thereto. The T-type calcium channel blocker herein is preferably selected from the group consisting of mibefradil, tetramethrin, ethosuximide, zonisamide, phyenytoin, SUN-N8075, efonidipine, trivalent metal ions selected from the group consisting of Y³⁺, La³⁺, Ce³⁺, Nd³⁺, Gd³⁺, Ho³⁺, Er³⁺, and Yb³⁺, Ni²⁺ U-92032 (7-[[4-[bis(4-fluorophenyl)methyl]-1-piperazinyl]methyl]-2-[(2-hydroxyethyl)amino]4-(1-methylethyl)-2,4,6-cycloheptatrien-1-one), penfluridol, fluspirilene, and valproate, and is more preferably ethosuximide, but not always limited thereto.

The present inventors confirmed through the preferred embodiments of the present invention that the animal model implanted with cobalt wire can be effectively used as the ADHD mouse model and the administration of ethosuximide, the T-type calcium channel blocker, can alleviate the symptoms of attention deficit disease. In addition, it was also confirmed that the composition comprising a T-type calcium channel blocker can be effectively used as the composition for the prevention and treatment of attention deficit disease.

A therapeutically effective dose of the T-type calcium channel blocker is determined by considering many related factors such as administration method, target area, and patient's condition, etc. For the administration to human, safety and efficiency have to be considered together. It is also possible to predict an effective dose for human based on the effective dose determined by animal test. Particularly, important factors to be considered for the determination of effective dose are described in the following references (Hardman and Limbird, eds., Goodman and Gilman's The Pharmacological Basis of Therapeutics, 10th ed.(2001), Pergamon Press; and E.W. Martin ed., Remington's Pharmaceutical Sciences, 18th ed.(1990), Mack Publishing Co.).

The composition of the present invention can include any generally used carrier, diluent, excipient, or a combination of at least two of those. The pharmaceutically acceptable carrier can be any carrier that is able to deliver the T-type calcium channel blocker in human body without limitation, which is exemplified by the compounds described in Merck Index, 13th ed., Merck & Co. Inc., such as saline, sterilized water, Ringer's solution, buffered saline, dextrose solution, maltodextrin solution, glycerol, ethanol, liposome and a mixture comprising one or more of those components. If necessary, a general additive such as antioxidant, buffer, and bacteriostatic agent can be additionally added. The composition of the present invention can be formulated in different forms including aqueous solutions, suspensions and emulsions for injection, pills, capsules, granules or tablets by mixing with diluents, dispersing agents, surfactants, binders and lubricants. A target organ specific antibody or other ligands can be mixed with one of the said carriers to be delivered to the target organ. The composition can further be prepared in suitable forms according to ingredients by following the method represented in Remington's Pharmaceutical Science (Mack Publishing Company, Easton PA, 18th, 1990).

The composition of the present invention can include one or more effective ingredients having the same or similar function to the T-type calcium channel blocker. The composition of the present invention can include the T-type calcium channel blocker by 0.0001 - 10 weight%, more preferably 0.001 - 1 weight% by the total weight of the composition.

The composition of the present invention can be administered orally or parenterally (for example, intravenous, hypodermic, peritoneal or local injection) but parenteral administration is preferred.

For formulations for parenteral administration, powders, granules, tablets, capsules, sterilized suspensions, liquids, water-insoluble excipients, suspensions, emulsions, syrups, suppositories, external use such as aerosols and sterilized injections can be prepared by the conventional method, and preferably skin external pharmaceutical compositions such as creams, gels, patches, sprays, ointments, plasters, lotions, liniments, pastes or cataplasms can be prepared, but not always limited thereto. For local administration, the composition can be prepared as nonhydrous or hydrous form according to clinical prescription. Water insoluble excipients and suspensions can contain, in addition to the active compound or compounds, propylene glycol, polyethylene glycol, vegetable oil like olive oil, injectable ester like ethylolate, etc. Suppositories can contain, in addition to the active compound or compounds, witepsol, macrogol, tween 61, cacao butter, laurin butter, glycerogelatin, etc.

Solid formulations for oral administration are powders, granules, tablets, capsules, soft capsules, and pills. Liquid formulations for oral administration are suspensions, solutions, emulsions, syrups and aerosols, and the above-mentioned formulations can contain various excipients such as wetting agents, sweeteners, aromatics and preservatives in addition to generally-used simple diluents such as water and liquid paraffin.

The effective dosage of the composition of the present invention can be determined according to weight, age, gender, health condition, diet, administration frequency, administration method, excretion and severity of a disease. The dosage is preferably 0.0001 mg ∼ 300 mg per day, and more preferably 0.001 mg ∼ 200 mg per day, and administration frequency is once a day or preferably a few times a day.

The present invention also provides a use of the ADHD mouse model of the present invention for the screening of an agent for the treatment of attention deficit disease.

In a preferred embodiment of the present invention, cobalt wire was implanted into the side of the right frontal lobe of a mouse and then the brain was sutured. In the mouse, cognitive impairment, hyperactivity, and prefrontal hyper-excitability, the characteristic symptoms of attention deficit disease, were observed. Such symptoms were not observed in the animal group implanted with copper wire or aluminum wire instead of cobalt wire or nothing. The above results suggest that the mouse implanted with cobalt wire of the present invention can be effectively used as the ADHD mouse model. Therefore, the present invention can further provide a use of the animal for the screening of an agent for the treatment of attention deficit disease since the symptoms of attention deficit disease was alleviated when it was administered with the T-type calcium channel blocker.

The present invention also provides a use of a T-type calcium channel blocker for the preparation of an agent for the treatment of attention deficit disease.

In a preferred embodiment of the present invention, specific brain wave, behavioral disorder, and cognitive impairment specifically observed in attention deficit disease were observed in the mouse model implanted with cobalt wire. When a T-type calcium channel blocker was administered to the ADHD mouse model, hyperactivity and abnormal prefrontal hyper-excitability were alleviated. Therefore, the T-type calcium channel blocker screened by using the ADHD mouse model of the present invention can be effectively used for the preparation of an agent for the treatment of attention deficit disease.

The present invention also provides a health food for the prevention and improvement of attention deficit disease comprising a T-type calcium channel blocker as an active ingredient.

The present invention also provides a use of a T-type calcium channel blocker for the preparation of a health food for the improvement of attention deficit disease.

In the health food for the prevention and improvement of attention deficit disease, the said attention deficit disease is preferably selected from the group consisting of attention deficit hyperactivity disorder, schizophrenia, frontal lobe epilepsy, and autism, but not always limited thereto. The T-type calcium channel blocker herein is preferably selected from the group consisting of mibefradil, tetramethrin, ethosuximide, zonisamide, phyenytoin, SUN-N8075, efonidipine, trivalent metal ions selected from the group consisting of Y³⁺, La³⁺, Ce³⁺, Nd³⁺, Gd³⁺, Ho³⁺, Er³⁺, and Yb³⁺, Ni²⁺, U-92032 (7-[[4-[bis(4-fluorophenyl)methyl]-1-piperazinyl]methyl]-2-[(2-hydroxyethyl)amino]4-(1-methylethyl)-2,4,6-cycloheptatrien-1-one), penfluridol, fluspirilene, and valproate, and is more preferably ethosuximide, but not always limited thereto.

The present inventors confirmed through the preferred embodiments of the present invention that the animal model implanted with cobalt wire can be effectively used as the ADHD mouse model and the administration of ethosuximide, the T-type calcium channel blocker, can alleviate the symptoms of attention deficit disease. In addition, it was also confirmed that the composition comprising a T-type calcium channel blocker can be effectively used as a health food for the prevention and improvement of attention deficit disease.

The T-type calcium channel blocker of the present invention can be provided as the food composition prepared by mixing the T-type calcium channel blocker with sitologically acceptable carriers.

The T-type calcium channel blocker of the present invention can be used as a food additive or a beverage additive. In that case, the T-type calcium channel blocker can be added as it is or as mixed with other food components according to the conventional method. The mixing ratio of active ingredients can be regulated according to the purpose of use (prevention, health enhancement or treatment).

For health and hygiene or regulating health condition, the T-type calcium channel blocker can be administered for a long time since the T-type calcium channel blocker has been proved to be very safe.

The food herein is not limited. For example, the T-type calcium channel blocker can be added to meats, sausages, breads, chocolates, candies, snacks, cookies, pizza, ramyuns, flour products, gums, dairy products including ice cream, soups, beverages, tea, drinks, alcohol drinks and vitamin complex, etc.

When the T-type calcium channel blocker is formulated as beverage, the beverage can additionally include various flavors or natural carbohydrates, etc, like other beverages. The natural carbohydrates above can be one of monosaccharides such as glucose and fructose, disaccharides such as maltose and sucrose, polysaccharides such as dextrin and cyclodextrin, and glucose alcohols such as xilytole, sorbitol and erythritol. The content of the natural carbohydrate is preferably 1 - 20 g and more preferably 5 - 12 g in 100 mℓ of the composition. Besides, natural sweetening agents such as thaumatin and stevia extract (ex, rebaudioside A, glycyrrhizin, etc), and synthetic sweetening agents such as saccharin and aspartame can be included as a sweetening agent.

In addition to the ingredients mentioned above, the food composition of the present invention can include in variety of nutrients, vitamins, minerals (electrolytes), flavors, coloring agents, enhancers (cheese, chocolate, etc), pectic acid and its salts, alginic acid and its salts, organic acid, protective colloidal viscosifiers, pH regulators, stabilizers, antiseptics, glycerin, alcohols, and carbonators which used to be added to soda, etc. The food composition of the present invention can also include fruit flesh addable to natural fruit juice, fruit beverages and vegetable beverages. All the mentioned ingredients can be added singly or together. The mixing ratio of those ingredients does not matter in fact, but in general, each can be added by 0.001 - 50 weight part by the total weight of the composition.

The present invention also provides a feed additive for the prevention and improvement of attention deficit disease comprising a T-type calcium channel blocker as an active ingredient.

In addition, the present invention provides a use of a T-type calcium channel blocker for the preparation of a feed additive for the improvement of attention deficit disease.

In the feed additive for the improvement of attention deficit disease, the said attention deficit disease is preferably selected from the group consisting of attention deficit hyperactivity disorder, schizophrenia, frontal lobe epilepsy, and autism, but not always limited thereto. The T-type calcium channel blocker herein is preferably selected from the group consisting of mibefradil, tetramethrin, ethosuximide, zonisamide, phyenytoin, SUN-N8075, efonidipine, trivalent metal ions selected from the group consisting of Y³⁺, La³⁺, Ce³⁺, Nd³⁺, Gd³⁺, Ho³⁺, Er³⁺, and Yb³⁺, Ni²⁺, U-92032 (7-[[4-[bis (4-fluorophenyl)methyl]-1-piperazinyl]methyl]-2-[(2-hydroxyethyl)amino]4-(1-methylethyl)-2,4,6-cycloheptatrien-1-one), penfluridol, fluspirilene, and valproate, and is more preferably ethosuximide, but not always limited thereto.

The present inventors confirmed through the preferred embodiments of the present invention that the animal model implanted with cobalt wire can be effectively used as the ADHD mouse model and the administration of ethosuximide, the T-type calcium channel blocker, can alleviate the symptoms of attention deficit disease. In addition, it was also confirmed that the composition comprising a T-type calcium channel blocker can be effectively used as a feed additive for the prevention and improvement of attention deficit disease.

The feed composition of the present invention can be prepared in the forms of fermented feed, formula feed, pellet, and silage by the conventional method. The amount of T-type calcium channel blocker added to the feed additive of the present invention is preferably 0.001 - 50 weight part by the total weight of the composition, but not always limited thereto.

Practical and presently preferred embodiments of the present invention are illustrative as shown in the following Examples, Experimental Examples and Manufacturing Examples.

However, it will be appreciated that those skilled in the art, on consideration of this disclosure, may make modifications and improvements within the spirit and scope of the present invention.

### Example 1: Construction of ADHD animal model

C57BL/6J mice at 10 - 20 weeks (BioModel System Park) were raised and treated according to standard animal care protocols of Korea Advanced Institute of Science and Technology (KAIST). Water and feed were provided freely and light/dark cycle was 12 hours.

Cobalt wire known to cause hypoxia was inserted in the right frontal lobe of the mouse to construct the ADHD animal model.

Particularly, to construct the ADHD mouse model, 0.2% avertin in 20 mg/ml of tribromoethanol was administered to the mouse by intraperitoneal injection (i.p.) to anesthetize it. The head of the mouse (B6 mouse at 10 - 20 weeks) was fixed on stereotaxic (David Kopf Instruments, Germany) and then cobalt wire (Alfa Aesar) was inserted therein by using holder, a part of stereotaxic. Cobalt wire (Alfa Aesar) of 500 *µ*m in the diameter was inserted radially (vertical direction to the cortical surface) in the location of anteroposteriorally 2.6 mm, laterally 1.8 mm, and ventrally 1.3 mm in the right dorsolateral frontal lobe.

For the record of EEG, epidural electrode was inserted in the right frontal lobe where the cobalt wire was inserted. Other electrodes were inserted in the left frontal lobe and temporal lobe. Particularly, the location for the insertion in the right frontal lobe was anteroposteriorally 2.8 mm and laterally 0.8 mm and the location in the left frontal lobe was anteroposteriorally 2.8 mm and laterally 0.8 mm. The grounding EEG was inserted in the occipital region of the scull.

In the case that EEG electrode was inserted together with cobalt wire, saturation was performed with dental cement. In the meantime, in the case that the behavioral test was performed without inserting EEG electrode, saturation was performed with surgical thread.

After three days of recovery, EEG signals and video images were recorded for 30 days. EGG signals were amplified (Grass model 7H polygraph, Grass Technologies, USA) and digitalized at the sampling rate of 500 Hz (DIGIDATA 1320A, Molecular Devices, USA). To obtain data, pClamp9.2 software (Molecular Devices, USA) was used.

### Example 2: Construction of T-type calcium channel knockout animal model

### <2-1> Construction of Cav3.1 T-type calcium channel knockout animal model

Cav3.1 T-type calcium channel knockout mouse model (Knockout, KO, -/-) was constructed by mating hetero mice having α1G T-type calcium channel only in the chromosome of one side (Neuron, 2001, Lack of the Burst Firing of Thalamocortical Relay Neurons and Resistance to Absence Seizures in Mice Lacking [alpha] 1G T-Type Ca2+ Channels). The knockout mouse prepared by such mating had no α1G T-type calcium channel at all in the whole body.

### <2-2> Construction of animal model in which α1G T-type calcium channel was partially removed in the mediodorsal thalamus

Lentivirus vector expressing shRNA targeting Cav3.1 T-type calcium channel was constructed. The synthesized double oligonucleotide (SEQ. ID. NO: 1; 5'- CGGAATTCCGGGAAGATCGTAGATA GCAAAttcaagagaTTTGCTATCTACGATCTTCTTTTTGATATCTAGACA - 3') was inserted in shLentisyn3.4G lentivirus vector (Macrogen, KOREA). The said shLentisyn3.4G lentivirus vector was designed to express shRNA from U6 promoter and to express enhanced green fluorescent protein (EGFP) from synapsin promoter.

The target sequence was not consistent with any mRNA in data base of National Center for Biotechnology Information except Cav3.1.

The scrambled version of Cav3.1 shRNA oligonucleotide had the sequence represented by SEQ. ID. NO: 2 (5'- C GGAATTCCGGGTAAGTGAA CTGACAAGAA ttcaagaga TTCTTGTCAGTTCACTTACT TTTTGATATCTAGACA -3') and was inserted in shLentisyn3.4G lentivirus vector (Macrogen, KOREA) to be used as the control. The said sequence showed no homology at all with any other mammal genes known up to date. The said recombinant lentivirus vector can be produced and concentrated commercially (Macrogen LentiVector Institute, Korea).

The present inventors used the lentivirus at the concentration of approximately 2×10⁶ transduction unit/mℓ. The solution containing Cav3.1 shRNA or the scrambled version used as the control was inserted into the ipsi-MD (ipsilateral MD thalamus, indicating that the virus was inserted in the right side of the mediodorsal thalamus) by using Nanofil 33G blunt needle and Nanofil syringe (World Precision Instruments, USA).

After 7 days from the virus transfection, cobalt wire and epidural electrode were inserted as described hereinbefore. EEG signals were recorded for 30 days along with video monitoring.

### Experimental Example 1: Observation of brain wave of ADHD animal model

To investigate whether or not the animal model constructed by inserting cobalt wire in the right frontal lobe in Example 1 could be used as the ADHD animal model, the inventors observed ADHD-specific brain wave.

For the measurement of brain wave (electroencephalogram, EEG) of the said animal model, EEG electrode was inserted in the right and left frontal lobes and the right and left temporal lobes, followed by observation of brain wave for 30 days. EEG electrode was inserted at the same time with cobalt wire. Like cobalt wire insertion, EEG electrode was inserted by using stereotaxic in the coordinate calculated.

Particularly, cobalt wire (Alfa Aesar) of 500 *µ*m in diameter was inserted radially (vertical direction to the cortical surface) in the location of anteroposteriorally 2.6 mm, laterally 1.8 mm, and ventrally 1.3 mm in the right dorsolateral frontal lobe. For the record of EEG, epidural electrode was inserted in the right frontal lobe where the cobalt wire was inserted. Other electrodes were inserted in the left frontal lobe and temporal lobe frontal lobe. Precisely, the location for the insertion in the right frontal lobe was anteroposteriorally 2.8 mm and laterally 0.8 mm and the location in the left frontal lobe was anteroposteriorally 2.8 mm and laterally 0.8 mm. The grounding EEG was inserted in the occipital of the scull.

After 3 - 4 days of recovery after the insertion of cobalt wire and EEG electrode, EEG signals and video images were recorded.

10 C57BL/6J mice that spent 4 days for recovery after the insertion of cobalt wire and EEG electrode were put in EEG changer (square bottomed box), in which they were free to move. EEG signals were amplified by using Grass model 7H polygraph (Grass Technologies, USA), followed by digitalization at the sampling rate of 500 Hz with DIGIDATA 1320A (Molecular Devices, USA). Then, date was obtained by using pClamp9.2 software (Molecular Devices, USA).

As a result, as shown in Figure 2, brain wave with low frequency was increased in the right frontal lobe only in the ADHD animal model implanted with cobalt wire. After theta oscillation of 1 - 7 Hz was detected first, spike type brain wave of 1 ∼ 4 Hz was began to be observed. Such spike type brain wave was not observed in the right frontal lobe where cobalt wire was not inserted (Figure 2).

### Experimental Example 2: Measurement of behavioral changes and cognitive function changes in ADHD animal model

To confirm if the animal model constructed in Example 1 by inserting cobalt wire in the right frontal lobe could be used as the ADHD animal model, the present inventors tried to observe ADHD-specific behavior.

The observation time point was set by the duration of cobalt wire insertion, such as 6 days (n=5), 14 days (n=5), and 28 days (n=9), and then behavior of each group was observed and compared with that of the control mouse group not inserted with cobalt wire (n=9). The mice were carefully placed in the open-field test kit (square bottomed acryl box, 40x40x 50 cm). Then, the distances that the mice traveled during 1 and 1/2 hour cycle were monitored at 5 minute intervals by digital video recording. The open-field test was performed between 18:00 to 22:00. EthoVision (Noduls, USA) was used for video image analysis.

Behavioral analysis was performed based on the open-field test (40x40x50 cm). As a result, as shown in Figure 3A, the distance that the ADHD animal model traveled increased as the insertion period of cobalt wire increased. This distance increase was significant compared with that of the control group not inserted with cobalt wire (Figure 3A).

In addition, traces of behavior of the ADHD mouse model that spent 28 days after the cobalt wire insertion were analyzed by using EthoVision (Noduls, USA). As a result, such stereotypy that the mouse was turning in one direction repeatedly was observed in the mouse implanted with cobalt wire (Figure 3B).

### Experimental Example 3: Measurement of cognitive function changes in ADHD animal model

To confirm whether or not the animal model constructed in Example 1 by inserting cobalt wire in the right frontal lobe could be effectively used as the ADHD animal model, the present inventors performed conditioned fear learning test to see if cognitive impairment, which is usually observed in hyperactivity disorder, might be detected therein.

To perform conditioned fear learning test, electric shock with a specific sound was given repeatedly to the mouse on the first day in order to make the mouse remember the fear. On the second day, two different tests were performed. First, it was tested whether or not the mouse could remember the space where it got the electric shock (context). Second, it was tested whether or not the mouse could remember the sound (cue). To test memory, freezing time of the mouse was measured. When a mouse feels fear, it does not move. Therefore, if the mouse remembered the fear, the freezing time would be longer. On the contrary, if the mouse did not remember the fear, the freezing time would be shorter. Cognitive function of the mouse implanted with cobalt wire was investigated by measuring such freezing time.

As a result, as shown in Figure 3C, cognitive impairment was observed in the animal model implanted with cobalt wire. That is, in the mouse inserted with cobalt wire, freezing time was significantly reduced in both context and cue tests, suggesting that the mouse implanted with cobalt wire did not remember fear by the electric shock.

### Experimental Example 4: Changes in brain wave of ADHD animal model by T-type calcium channel blocker <4-1> Changes in brain wave of ADHD animal model by ethosuximide

As observed in <Experimental Example 1>, electric signal with low frequency was increased characteristically in the frontal lobe of the ADHD animal model of the present invention. To relive such prefrontal hyper-excitability, ethosuximide known as one of T-type calcium channel blockers (Huguenard, 2002) was administered to the hyperactivity disorder mouse model.

### <4-2> Changes in brain wave of ADHD animal model by ethosuximide

As observed in <Experimental Example 1>, electric signal with low frequency was increased characteristically in the frontal lobe of the ADHD animal model of the present invention. In this example, T-type calcium channel blockers such as ethosuximide (Huguenard, 2002), zonisamide (ZNS; Kito et al., 1996), and phyenytoin (PHT; Lacinova et al., 2000) were treated to the hyperactivity disorder mouse model.

Particularly, 150 mg/kg of ethosuximide (Sigma-Aldrich, USA) was administered in the abdominal cavity of the ADHD animal model implanted with cobalt wire 7 - 9 days earlier, followed by observation of the changes in brain wave for one hour. The mice were placed in the test room having square bottom where they could move freely and brain waves were being recorded from 30 minutes before the ethosuximide administration. The number of spikes was measured before and after the treatment of ethosuximide. As a result, when ethosuximide was administered, the number of spikes observed in the frontal lobe was significantly reduced, compared with before the treatment. Therefore, it was confirmed that ethosuximide could alleviate electric excitation in the frontal lobe, suggesting that ethosuximide could relieve the symptom of hyperactivity disorder.

60 mg/kg of zonisamide (Sigma-Aldrich, USA) and 100 mg/kg of phyenytoin (Sigma-Aldrich, USA) were administered respectively in the abdominal cavity of the ADHD animal model implanted with cobalt wire 7 ~ 9 days earlier, followed by observation of the changes in brain wave for one hour. The mice were placed in the test room having square bottom where they could move freely and brain waves were being recorded from 30 minutes before the ethosuximide administration.

The number of spikes was measured before and after the treatment of zonisamide or phyenytoin. As a result, as shown in Figure 4, when zonisamide and phyenytoin, the T-type calcium channel blockers, were administered to the ADHD animal model of the present invention respectively, spikes observed in the frontal lobe were significantly reduced, compared with before the treatment (P<0.05) (Figure 4).

### Experimental Example 5: Changes in brain wave by other injected materials except cobalt wire

To investigate changes in brain wave pattern by other metals except cobalt wire, the present inventors implanted copper wire and aluminum wire by the same manner as described hereinabove for the insertion of cobalt wire.

Particularly, the head of mouse (B6 mouse at 10 ~ 20 weeks) was fixed on stereotaxic and then cobalt wire (Alfa Aesar) was inserted therein by using holder, a part of stereotaxic.

Cobalt wire (Alfa Aesar) of 500 *µ*m in diameter was inserted radially (vertical direction to the cortical surface) in the location of anteroposteriorally 2.6 mm, laterally 1.8 mm, and ventrally 1.3 mm in the right dorsolateral frontal lobe. For the record of EEG, epidural electrode was inserted in the right frontal lobe where the cobalt wire was inserted. Other electrodes were inserted in the left frontal lobe and temporal lobe frontal lobe. Particularly, the location for the insertion in the right frontal lobe was anteroposteriorally 2.8 mm and laterally 0.8 mm and the location in the left frontal lobe was anteroposteriorally 2.8 mm and laterally 0.8 mm. The grounding EEG was inserted in the occipital region of the scull.

The location of insertion of each copper wire and aluminum wire was same as that of cobalt wire. That is copper wire or aluminum wire was inserted radially (vertical direction to the cortical surface) in the location of anteroposteriorally 2.6 mm, laterally 1.8 mm, and ventrally 1.3 mm in the right dorsolateral frontal lobe. EEG electrode was inserted together and sutured with dental cement.

As a result, as shown in Figure 5, in the group inserted with copper wire or aluminum wire, spike type brain wave was not observed, unlike in the group implanted with cobalt wire. This result was similar to that of the control not inserted with anything (Figure 5). Therefore, it was confirmed that the mouse model inserted with nothing could be used as the control.

### Experimental Example 6: Observation of brain wave and behavior of Cav3.1 T-type calcium channel knockout animal model

### <6-1> Observation of brain wave in the frontal lobe of Cav3.1 T-type calcium channel knockout animal model

The present inventors investigated the mechanism of spike generation in the frontal lobe by using the Cav3.1 T-type calcium channel knockout animal model of <Example 2-1>.

After the Cav3.1 T-type calcium channel knockout mouse was born, cobalt wire and epidural electrode for EEG recording were inserted therein when the mouse was at 15 - 20 weeks, by the same method described in <Example 1>, followed by observation of electroencephalogram (EEG) for 30 days.

As a result, as shown in Figure 6A, in the α1G T-type calcium channel (known as important to generate brain wave in the mediodorsal thalamus) knockout animal model, spikes with low frequency were significantly reduced in the frontal lobe after 6 ~ 7 days from the cobalt wire insertion. Brain wave with low frequency observed in the frontal lobe of the α1G T-type calcium channel knockout animal model was significantly reduced, compared with that of the control (T test, P<0.05). Therefore, it was confirmed that α1G T-type calcium channel played an important role in the development of frontal lobe related disease including ADHD (Figure 6A).

### <6-2> Observation of behavioral changes of Cav3.1 T-type calcium channel knockout animal model

The present inventors investigated the mechanism of spike generation in the frontal lobe by using the Cav3.1 T-type calcium channel knockout animal model of <Example 2-1>.

After the Cav3.1 T-type calcium channel knockout mouse was born, cobalt wire and epidural electrode for EEG recording were inserted therein when the mouse was at 15 - 20 weeks, by the same method described in <Example 1>, followed by observation of electroencephalogram (EEG) for 30 days.

The mice were carefully placed in the open-field test kit (square bottomed acryl box, 40x40x50 cm). Then, the distances that the mice traveled during 1 and 1/2 hour cycle were monitored at 5 minute intervals by digital video recording. The open-field test was performed between 18:00 to 22:00. EthoVision (Noduls, USA) was used for video image analysis.

Behavioral analysis was performed based on the open-field test (40x40x50 cm). As a result, as shown in Figure 6B, the α1G T-type calcium channel knockout animal model demonstrated longer moving distance, after 6 - 7 days from the cobalt wire implant, compared with that of the control. Such increased movement was significant compared with that of the control in which α1G T-type calcium channel was not eliminated (Figure 6B).

### Experimental Example 7 Observation of brain wave and behavior of α1G T-type calcium channel knockout animal model

### <7-1> Observation of brain wave in the mediodorsal thalamus of α1G T-type calcium channel knockout animal model

To suppress the expression of α1G T-type calcium channel in the mediodorsal thalamus, the present inventors transfected the mouse locally in the mediodorsal thalamus with the lentivirus vector expressing shRNA targeting Cav3.1 T-type calcium channel of <Example 2-2>. The present inventors then investigated the mechanism of spike generation in the mediodorsal thalamus using the animal model in which α1G T-type calcium channel was partially removed in the mediodorsal thalamus.

7 days after the transfection with the lentivirus vector expressing shRNA targeting Cav3.1 T-type calcium channel in the mediodorsal thalamus, cobalt wire and epidural electrode for EEG recording were inserted by the same manner as described in <Example 1> to investigate characteristic behavior of hyperactivity disorder.

As a result, as shown in Figure 7, in the α1G T-type calcium channel (known as important to generate brain wave in the mediodorsal thalamus) knockout animal model, spikes with low frequency were observed in the frontal lobe. Brain wave with low frequency observed in the frontal lobe of the α1G T-type calcium channel knockout animal model was significantly reduced, compared with that of the control (T test, P<0.05). Therefore, it was confirmed that α1G T-type calcium channel played an important role in the development of frontal lobe related disease including ADHD (Figure 7).

### <7-2> Observation of behavioral changes of α1G T-type calcium channel knockout animal model

To suppress the expression of α1G T-type calcium channel in the mediodorsal thalamus, the present inventors transfected the mouse locally in the mediodorsal thalamus with the lentivirus vector expressing shRNA targeting Cav3.1 T-type calcium channel of <Example 2-2>. The present inventors then investigated the mechanism of spike generation in the mediodorsal thalamus using the animal model in which α1G T-type calcium channel was partially removed in the mediodorsal thalamus.

7 days after the transfection with the lentivirus vector expressing shRNA targeting Cav3.1 T-type calcium channel in the mediodorsal thalamus, cobalt wire and epidural electrode for EEG recording were inserted by the same manner as described in <Example 1> to investigate characteristic behavior of hyperactivity disorder.

The mice were carefully placed in the open-field test kit (square bottomed acryl box, 40x40x50 cm). Then, the distances that the mice traveled during 1 and 1/2 hour cycle were monitored at 5 minute intervals by digital video recording. The open-field test was performed between 18:00 to 22:00. EthoVision (Noduls, USA) was used for video image analysis.

Behavioral analysis was performed based on the open-field test (40x40x50 cm). As a result, as shown in Figure 8A, the α1G T-type calcium channel knockout animal model demonstrated longer moving distance, compared with that of the control. Such increased movement was significant compared with that of the control in which α1G T-type calcium channel was not eliminated (Figure 8A). This result is described in the graph of Figure 8B.

The Manufacturing Examples of the composition for the present invention are described hereinafter.

### Manufacturing Example 1: Preparation of pharmaceutical formulations

### <1-1> Preparation of powders

| | |
|---|---|
| Ethosuximide | 2 g |
| Lactose | 1 g |

Powders were prepared by mixing all the above components, which were filled in airtight packs according to the conventional method for preparing powders.

### <1-2> preparation of tablets

| | |
|---|---|
| Ethosuximide | 100mg |
| Corn starch | 100mg |
| Lactose | 100mg |
| Magnesium stearate | 2mg |

Tablets were prepared by mixing all the above components by the conventional method for preparing tablets.

### <1-3> Preparation of capsules

| | |
|---|---|
| Ethosuximide | 100mg |
| Corn starch | 100mg |
| Lactose | 100mg |
| Magnesium stearate | 2mg |

Capsules were prepared by mixing all the above components, which were filled in gelatin capsules according to the conventional method for preparing capsules.

### <1-4> Preparation of pills

| | |
|---|---|
| Ethosuximide | 1 g |
| Lactose | 1.5 g |
| Glycerin | 1 g |
| Xylitol | 0.5 g |

Pills were prepared by mixing all the above components according to the conventional method for preparing pills. Each pill contained 4 g of the mixture.

### <1-5> Preparation of granules

| | |
|---|---|
| Ethosuximide | 150 mg |
| Soybean extract | 50 mg |
| Glucose | 200 mg |
| Starch | 600 mg |

All the above components were mixed, to which 100 mg of 30% ethanol was added. The mixture was dried at 60°C and the prepared granules were filled in packs.

### Manufacturing Example 2: Preparation of food

### <2-1> Preparation of snacks and flour foods

0.5 - 5.0 weight part of the extract of Example <1-1> was added to the flour. Health enhancing foods such as bread, cake, cookies, crackers and noodles were prepared with the flour mixture according to the conventional method.

### <2-2> Preparation of dairy products

5 - 10 weight part of the extract of Example <1-1> was added to milk. Health enhancing dairy products such as butter and ice cream were prepared with the milk mixture according to the conventional method.

### <2-7> Preparation of Sun-Sik

Brown rice, barley, glutinous rice and Yulmu (Job's tears) were gelatinized according to the conventional method, dried and pulverized to obtain 60-mesh powders.

Black soybean, black sesame and wild sesame were steamed and dried according to the conventional method and pulverized to obtain 60-mesh powders.

The extract of Example <1-1> was concentrated under reduced pressure, spray-dried and pulverized to obtain 60-mesh dry powders.

Sun-Sik was prepared by mixing the dry powders of the grains, seeds and the extract of Example <1-1> according to the below ratio.

Grains (brown rice: 30 weight part, Yulmu: 15 weight part, barley: 20 weight part),

Seeds (wild sesame: 7 weight part, black soybean: 8 weight part, black sesame: 7 weight part),

Dry powders of the compound isolated from the extract of Example <1-1> (3 weight part),

*Ganoderma lucidum* (0.5 weight part),

*Rehmannia glutinosa* (0.5 weight part)

Manufacturing Example 3: Preparation of beverages

| | |
|---|---|
| Extract of Example <1-1> | 1000 mg |
| Citric acid | 1000 mg |
| Oligosaccharide | 100 g |
| Maesil (Prunus mume) Extract | 2 g |
| Taurine | 1 g |
| Purified water | up to 900 ml |

The above constituents were mixed according to the conventional method for preparing health beverages. The mixture was heated at 85°C for 1 hour with stirring and then filtered. The filtrate was loaded in 2 liter sterilized containers, which were sealed and sterilized again, stored in a refrigerator until they would be used for the preparation of a composition for health beverages.

The constituents appropriate for favorite beverages were mixed according to the preferred mixing ratio but the composition ratio can be adjusted according to regional and national preferences, etc.

### INDUSTRIAL APPLICABILITY

As explained hereinbefore, the Attention deficit and hyperactivity disorder(ADHD) animal model constructed by implanting cobalt wire of the present invention demonstrated hyperactivity and abnormal prefrontal hyper-excitability, which are the characteristic symptoms of attention deficit disease, and these symptoms could be alleviated by the administration of ethosuximide. Therefore, ethosuximide can be effectively used as an active ingredient of the composition for the prevention and treatment of attention deficit disease showing hyperactivity symptoms.

Those skilled in the art will appreciate that the conceptions and specific embodiments disclosed in the foregoing description may be readily utilized as a basis for modifying or designing other embodiments for carrying out the same purposes of the present invention. Those skilled in the art will also appreciate that such equivalent embodiments do not depart from the spirit and scope of the invention as set forth in the appended Claims.

## Claims

1. An Attention deficit and hyperactivity disorder(ADHD) mouse model constructed by the following steps:
1) implanting cobalt wire in the side of the right frontal lobe of mouse brain;
2) suturing the mouse brain after completing the implant of cobalt wire; and
3) selecting those mice showing attention deficit.

2. The ADHD mouse model according to claim 1, wherein the cobalt wire is 400 ~ 500 µm in diameter.

3. The ADHD mouse model according to claim 1, wherein the cobalt wire is inserted radially in the location of anteroposteriorally 2.0 - 3.0 mm, laterally 1.5 - 2.5 mm, and ventrally 1.0 ~ 2.0 mm in the right dorsolateral frontal lobe.

4. A method for constructing ADHD mouse model comprising the following steps:
1) implanting cobalt wire in the side of the right frontal lobe of mouse brain;
2) suturing the mouse brain after completing the implant of cobalt wire; and
3) selecting those mice showing attention deficit.

5. A screening method of an agent for the treatment of attention deficit disease comprising the following steps:
1) administering test compounds or compositions to the ADHD mouse model of claim 1;
2) measuring brain wave of the ADHD mouse model administered with test compounds or compositions of step 1); and
3) selecting the test compound or the composition demonstrating to be effective in reducing abnormal electroencephalogram(EEG) spikes in the ADHD mouse model of step 2) by comparing the control group not treated with test compounds or compositions.

6. The screening method of an agent for the treatment of attention deficit disease according to claim 5, wherein the attention deficit disease is preferably selected from the group consisting of attention deficit hyperactivity disorder, schizophrenia, frontal lobe epilepsy, and autism.

7. A screening method of an agent for the treatment of attention deficit disease comprising the following steps:
1) administering test compounds or compositions to the ADHD mouse model of claim 1;
2) investigating behavioral disorder or cognitive function of the ADHD mouse model administered with test compounds or compositions of step 1); and
3) selecting the test compound or the composition demonstrating to be effective in recovery of behavioral disorder or cognitive function by comparing the brain wave of the ADHD mouse model of step 2) with that of the control group not treated with test compounds or compositions.

8. The screening method of an agent for the treatment of attention deficit disease according to claim 7, wherein the behavioral disorder is the stereotypy.

9. The screening method of an agent for the treatment of attention deficit disease according to claim 7, wherein the cognitive function is measured by conditioned fear learning test.

10. A composition for the prevention and treatment of attention deficit disease comprising a T-type calcium channel blocker as an active ingredient.

11. The composition for the prevention and treatment of attention deficit disease according to claim 10, wherein the T-type calcium channel is α1G T-type calcium channel.

12. The composition for the prevention and treatment of attention deficit disease according to claim 10, wherein the attention deficit disease is preferably selected from the group consisting of attention deficit hyperactivity disorder, schizophrenia, frontal lobe epilepsy, and autism.

13. The composition for the prevention and treatment of attention deficit disease according to claim 10, wherein the T-type calcium channel blocker is preferably selected from the group consisting of mibefradil, tetramethrin, ethosuximide, zonisamide, phyenytoin, SUN-N8075, efonidipine, trivalent metal ions selected from the group consisting of Y³⁺, La³⁺, Ce³⁺, Nd³⁺, Gd³⁺, Ho³⁺, Er³⁺, and Yb³⁺, Ni²⁺, U-92032 (7-[[4-[bis(4-fluorophenyl)methyl]-1-piperazinyl]methyl]-2-[(2-hydroxyethyl)amino]4-(1-methylethyl)-2,4,6-cycloheptatrien-1-one), penfluridol, fluspirilene, and valproate.

14. The composition for the prevention and treatment of attention deficit disease according to claim 10, wherein the T-type calcium channel blocker is preferably selected from the group consisting of ethosuximide, zonisamide, and phyenytoin.

15. A health food for the prevention and improvement of attention deficit disease comprising a T-type calcium channel blocker as an active ingredient.

16. The health food for the prevention and improvement of attention deficit disease according to claim 15, wherein the attention deficit disease is preferably selected from the group consisting of attention deficit hyperactivity disorder, schizophrenia, frontal lobe epilepsy, and autism.

17. The health food for the prevention and improvement of attention deficit disease according to claim 15, wherein the T-type calcium channel blocker is preferably selected from the group consisting of mibefradil, tetramethrin, ethosuximide, zonisamide, phyenytoin, SUN-N8075, efonidipine, trivalent metal ions selected from the group consisting of Y³⁺, La³⁺, Ce³⁺, Nd³⁺, Gd³⁺, Ho³⁺, Er³⁺, and Yb³⁺, Ni²⁺, U-92032 (7-[[4-[bis(4-fluorophenyl)methyl]-1-piperazinyl]methyl]-2-[(2-hydroxyethyl)amino]4-(1-methylethyl)-2,4,6-cycloheptatrien-1-one), penfluridol, fluspirilene, and valproate.

18. The health food for the prevention and improvement of attention deficit disease according to claim 15, wherein the T-type calcium channel blocker is preferably selected from the group consisting of ethosuximide, zonisamide, and phyenytoin.

19. A feed additive for the prevention and improvement of attention deficit disease comprising a T-type calcium channel blocker as an active ingredient.

20. The feed additive for the prevention and improvement of attention deficit disease according to claim 19, wherein the attention deficit disease is preferably selected from the group consisting of attention deficit hyperactivity disorder, schizophrenia, frontal lobe epilepsy, and autism.

21. The feed additive for the prevention and improvement of attention deficit disease according to claim 19, wherein the T-type calcium channel blocker is preferably selected from the group consisting of mibefradil, tetramethrin, ethosuximide, zonisamide, phyenytoin, SUN-N8075, efonidipine, trivalent metal ions selected from the group consisting of Y³⁺, La³⁺, Ce³⁺, Nd³⁺, Gd³⁺, Ho³⁺, Er³⁺, and Yb³⁺, Ni²⁺, U-92032 (7-[[4-[bis(4-fluorophenyl)methyl]-1-piperazinyl]methyl]-2-[(2-hydroxyethyl)amino]4-(1-methylethyl)-2,4,6-cycloheptatrien-1-one), penfluridol, fluspirilene, and valproate.

22. The feed additive for the prevention and improvement of attention deficit disease according to claim 19, wherein the T-type calcium channel blocker is ethosuximide.

23. A use of the mouse of claim 1 for the screening of an agent for the treatment of attention deficit disease.

24. A use of the T-type calcium channel blocker for the preparation of an agent for the treatment of attention deficit disease.

25. A use of the T-type calcium channel blocker for the preparation of a health food for the improvement of attention deficit disease.

26. A use of the T-type calcium channel blocker for the preparation of a feed additive for the improvement of attention deficit disease.

27. A use of the T-type calcium channel blocker according to one of claim 23 - claim 26, wherein the T-type calcium channel blocker is preferably selected from the group consisting of mibefradil, tetramethrin, ethosuximide, zonisamide, phyenytoin, SUN-N8075, efonidipine, trivalent metal ions selected from the group consisting of Y³⁺, La³⁺, Ce³⁺, Nd³⁺, Gd³⁺, Ho³⁺, Er³⁺, and Yb³⁺, Ni²⁺, U-92032 (7-[[4-[bis(4-fluorophenyl)methyl]-1-piperazinyl]methyl]-2-[(2-hydroxyethyl)amino]4-(1-methylethyl)-2,4,6-cycloheptatrien-1-one), penfluridol, fluspirilene, and valproate.
